# EUROPÄISCHE PATENTSCHRIFT

(11) **EP 2 320 797 B1**
(45) Veröffentlichungstag und Bekanntmachung des Hinweises auf die Patenterteilung: **19.04.2017**
(21) Anmeldenummer: 09757464.4
(22) Anmeldetag: 29.05.2009
(51) Int. Cl.: A61B 5/15

(54) **STECHVORRICHTUNG FÜR EINE BLUTPROBENENTNAHME**
PRICKING DEVICE FOR TAKING A BLOOD SAMPLE
DISPOSITIF DE PIQÛRE PERMETTANT LE PRÉLÈVEMENT D'UN ÉCHANTILLON DE SANG

(30) Priorität: 06.06.2008 DE 102008027272; 08.08.2008 DE 102008037082
(43) Veröffentlichungstag der Anmeldung: 18.05.2011
(73) Patentinhaber: Gerresheimer Regensburg GmbH, 93051 Regensburg (DE)
(72) Erfinder: BUTZ, Marion, 93049 Regensburg (DE); KNIE, Andreas, 93051 Regensburg (DE); VACLAV, Vojan, 32300 Plzen (CZ)
(74) Vertreter: Hannke, Christian
(86) Internationale Anmeldenummer: PCT/EP2009/056597
(87) Internationale Veröffentlichungsnummer: WO 2009/147082

(56) Entgegenhaltungen:
- EP-A- 0 898 936
- EP-A- 1 090 584
- WO-A-2005/077275
- US-A- 5 527 334
- US-A1- 2005 131 441
- US-B1- 6 221 089

## Beschreibung

Die Erfindung wird durch die Ansprüche definiert. Alle anderen Ausführungsformen sind lediglich beispielhaft.

Die Erfindung betrifft eine Stechvorrichtung für eine Blutprobenentnahme mit einer verlagerbaren Halteeinrichtung für ein Stechmittel, mit Mitteln zum Antreiben der verlagerbaren Halteeinrichtung und mit einer Auslöseeinrichtung zum Auslösen einer Stechbewegung des Stechmittels, bei welcher nach einem manuellen Betätigen der Auslöseeinrichtung die verlagerbare Halteeinrichtung mittels einer Federkraft einer Antriebsfeder der Antriebsmittel axial verlagert werden kann.

Gattungsgemäße Stechvorrichtungen sind aus dem Stand der Technik gut bekannt und haben sich zum Entnehmen einer Blutprobe, beispielsweise hinsichtlich einer Blutzuckermessung, gut bewährt. Oftmals sind solche Stechvorrichtungen nur für den einmaligen Gebrauch vorgesehen. Um derartige Stechvorrichtungen jedoch auch mehrmals verwenden zu können, ist es erforderlich, beispielsweise Antriebsfedern innerhalb der Stechvorrichtung für einen erneuten Einstechvorgang neu zu spannen.

Es ist Aufgabe der Erfindung gattungsgemäße Stechvorrichtungen derart weiterzuentwickeln, dass auch deren mehrmaliger Gebrauch angenehmer zu handhaben ist.

Die Aufgabe wird von einer Stechvorrichtung für eine Blutprobenentnahme mit einer verlagerbaren Halteeinrichtung für ein Stechmittel, mit Mitteln zum Antreiben der verlagerbaren Halteeinrichtung und mit einer Auslöseeinrichtung zum Auslösen einer Stechbewegung des Stechmittels gelöst, bei welcher nach einem manuellen Betätigen der Auslöseeinrichtung die verlagerbare Halteeinrichtung mittels einer Federkraft einer Antriebsfeder der Antriebsmittel axial verlagert werden kann, wobei die Stechvorrichtung sich durch eine Spanneinrichtung zum Spannen der Antriebsfeder auszeichnet, wobei weiter die Spanneinrichtung einen Spannmechanismus umfassend Mittel zum axialen Arretieren der verlagerbaren Halteeinrichtung während des Spannens der Antriebsfeder und umfassend Mittel zum Verrichten von Arbeit während des Spannens der Antriebsfeder aufweist.

Auf Grund der Spanneinrichtung mit einem derartigen Spannmechanismus gelingt es wesentlich einfacher und betriebssicherer die Stechvorrichtung erneut in einen gespannten und betriebsbereiten Zustand zu überführen, um eine weitere Blutprobenentnahme durchführen zu können.

Es versteht sich, dass die Spanneinrichtung konstruktiv auf unterschiedliche Weise umgesetzt werden kann. Vorliegend ist es wesentlich, dass der Spannmechanismus der Spanneinrichtung einerseits Mittel zum axialen Arretieren der verlagerbaren Halteeinrichtung umfasst, um vorteilhafter Weise während des Spannens der Antriebsfeder ein unbeabsichtigtes Verlagern der Halteeinrichtung unterbinden zu können. Hierdurch kann insbesondere verhindert werden, dass das Stechmittel kritisch aus einem Gehäuse der Spannvorrichtung heraus bewegt werden kann, ohne eine gezielte Blutprobenentnahme vornehmen zu wollen. Andererseits ist es wesentlich, dass der Spannmechanismus Mittel zum Verrichten von Arbeit umfasst, um vorteilhaft einen Spannvorgang kräftemäßig unterstützten zu können, wodurch das Spannen der Antriebsfeder wesentlich erleichtert wird.

Als Stechmittel können vorliegend Gebilde, wie Nadeln, eingesetzt werden, welche nicht nur einen runden Querschnitt sondern auch einen rechteckigen Querschnitt bzw. eine schneidförmige Spitze aufweisen.

Der Begriff "Antriebsfeder" beschreibt im Sinne der Erfindung jegliche Gebilde, mittels welcher im Wesentlichen eine Stechbewegung des Stechmittels initiiert werden kann. Hierzu muss insbesondere die verlagerbare Halteeinrichtung beschleunigt werden, wobei das Stechmittel an sich die verlagerbare Halteeinrichtung bilden könnte. Eine Antriebsfeder kann als Blattfeder etwa aus Kunststoff bestehen. Oder ein elastischer Gummizug kann im Sinne der vorliegenden Antriebsfeder verwendet werden. Bevorzugt wird jedoch eine Antriebsfeder aus Metall in Gestalt einer Schenkelfeder, da diese dauerhaft eine im Wesentlichen gleichbleibende Federkraft bereitstellen kann.

Eine bevorzugte Ausführungsvariante sieht vor, dass die Spanneinrichtung einen axial verschiebbaren Spannschlitten umfasst. Mittels des axial verschiebbaren Spannschlittens können alle insbesondere an einem Spannvorgang der Antriebsfeder wesentlich beteiligten Komponenten aus einer Ruheposition, welche die Komponenten nach einer erfolgten Stechbewegung des Stechmittels eingenommen haben, zielgerichtet und zueinander koordiniert in eine Startposition überführt werden.

Weist der axial verschiebbare Spannschlitten einen Schiebesupport für ein Steuerkurvenbahnelement und/oder für eine Antriebsfederlagerung auf, können diese Komponenten besonders vorteilhaft manuell gegenüber weiteren Komponenten der Stechvorrichtung derart verschoben werden, dass sie baulich besonders einfach wieder gemäß eines betriebsbereiten Zustandes der Stechvorrichtung positioniert werden können. Es versteht sich, dass weitere Komponenten der Stechvorrichtung mittels des verschiebbaren Spannschlittens vorteilhaft positioniert werden können, wenn sich dies als vorteilhaft erweisen sollte.

Des Weiteren ist es vorteilhaft, wenn die Mittel zum Verrichten von Arbeit Federelemente, vorzugsweise zwei Spiralfedern, aufweisen. Die Mittel zum Verrichten von Arbeit können vorliegend von jeglichen Einrichtungen realisiert werden, welche unterstützend beim Spannen der Antriebsfedern wirken können. Als ausreichend stark und besonders wartungsarm und langlebig haben sich Spiralfedern erwiesen, so dass vorliegend Spiralfedern als Mittel zum Verrichten von Arbeit herausragend gut eingesetzt werden können.

Eine weitere bevorzugte Ausführungsvariante sieht vor, dass die Mittel zum Verrichten von Arbeit einen Spannkopf der Spanneinrichtung federnd an einem Grundkörper der Stechvorrichtung lagern. Wie hinsichtlich der Ausführungsbeispiele aus der anliegenden Zeichnung noch beschrieben ist, können die Mittel zum Verrichten von Arbeit einen Spannkopf vorteilhaft an dem Gehäuse der Stechvorrichtung lagern, wobei die Mittel zum Verrichten von Arbeit zugleich mittels einer axialen Auslenkung des Spannkopfes derart vorgespannt werden können, dass mittels ihnen idealerweise zur Gänze die Antriebsfeder in einen gespannten Zustand überführt werden kann.

In diesem Zusammenhang ist es vorteilhaft, wenn die Mittel zum Verrichten von Arbeit eine Arbeitsfederkraft F_{D} und die Antriebsfeder eine Antriebsfederkraft F_{S} aufweisen, wobei die Arbeitsfederkraft F_{D} größer ist als die Antriebsfederkraft F_{S}, wodurch die Antriebsfeder besonders komfortabel gespannt werden kann.

Um die Arbeitsfederkraft F_{D} insbesondere von einem Schenkel einer Schenkelfeder als Antriebsfeder baulich einfach und relativ verlustarm auf weitere Komponenten der Antriebsmittel übertragen zu können, ist es vorteilhaft, wenn die Antriebsmittel ein Koppelglied zwischen der verlagerbaren Halteeinrichtung und einem Steuerkurvenbahnelement der Antriebsmittel umfassen. Das Koppelglied ist hierbei schwenkbar an der verlagerbaren Halteeinrichtung gelagert, so dass es mit seinem der Halteeinrichtungslagerung gegenüber liegenden Ende radial hinsichtlich einer axialen Verlagerungsachse der Halteeinrichtung ausgelenkt werden kann.

Damit das Koppelglied in einem definierten Bogenabschnitt geführt werden kann, ist es weiter vorteilhaft, wenn die Antriebsmittel eine Kurvenbahn, vorzugsweise eine bogenförmige Kurvenbahn, umfassen. Insbesondere kann mittels einer solchen Kurvenbahn das der Halteeinrichtungslagerung gegenüber liegende Ende des Koppelgliedes zielgerichtet geführt werden. Mittels der bogenförmigen Kurvenbahn kann konstruktiv besonders einfach eine translatorische Vorwärtsbewegung, eine maximale axiale Auslenkung und eine translatorische Rückwärtsbewegung des Stechmittels erzielt werden.

Umfassen die Antriebsmittel ein Steuerkurvenbahnelement, kann die Kurvenbahn vorteilhaft in eines der Antriebsmittel integriert werden.

Das Koppelglied kann entlang der Kurvenbahn mit hoher Präzision geführt werden, wenn ein Kurvenbahnfolger vorgesehen ist, der innerhalb einer Kulisse der Kurvenbahn der Antriebsmittel angeordnet ist. Der Kurvenbahnfolger kann hierbei in Gestalt eines Zapfens vorgesehen werden, welcher in eine gebogene Nut am Steuerkurvenbahnelement hineinragt.

Eine besonders vorteilhafte Ausführungsvariante sieht vor, dass der Kurvenbahnfolger einen Vorwärtstrieb aufweist, mittels welchem der Kurvenbahnfolger entlang der Kurvenbahn in eine erste Richtung bewegt werden kann. Ein solcher Vorwärtstrieb kann beispielsweise mittels der vorliegenden Antriebsfeder besonders kompakt verwirklicht sein.

Kumulativ oder alternativ ist es vorteilhaft, wenn der Kurvenbahnfolger einen Rückwärtstrieb aufweist, mittels welchem der Kurvenbahnfolger entlang der Kurvenbahn in eine zweite der ersten Richtung entgegengesetzte Richtung bewegt werden kann. Ein geeigneter Rückwärtstrieb kann schon mit den Mitteln zum Verrichten von Arbeit, insbesondere in Gestalt von Spiralfedern, geschaffen werden.

Durch den Aufbau der vorliegenden Stechvorrichtung, speziell im Hinblick auf das Zusammenwirken der einzelnen Antriebsmittel, kann insbesondere auch funktionssicher gewährleistet werden, dass eine nahezu immer gleiche Eindringtiefe und Verlaufsbahn des Stechmittels erreicht wird. Hierdurch kann ein Einstechschmerz beim Eindringen des Stechmittels in eine obere Hautschicht zum Entnehmen einer Blutprobe vorteilhaft reduziert werden. Insbesondere kann mittels des Aufbaus vorliegender Antriebsmittel ein unerwünschtes Nachschwingen des Stechmittels verhindert werden, wie es bei bekannten federgeführten Haltesystemen der Fall ist. Somit stechen die Stechmittel vorliegend vorteilhafter Weise nur einmal zu.

Weitere Vorteile, Ziele und Eigenschaften vorliegender Erfindung werden anhand nachfolgender Beschreibung anliegender Zeichnung erläutert, in welcher beispielhaft zwei Ausführungsbeispiele hinsichtlich einer Stechvorrichtung mit einer Spanneinrichtung dargestellt sind. Komponenten, welche in den Figuren wenigstens im Wesentlichen hinsichtlich ihrer Funktion übereinstimmen, können hierbei mit gleichen Bezugsziffern gekennzeichnet sein, wobei diese Komponenten nicht in allen Figuren beziffert und erläutert sein müssen.

Es zeigt
- Figur 1: schematisch eine erste geschnittene Ansicht eines ersten Ausführungsbeispiels einer Stechvorrichtung in einem gespannten und einsatzbereiten Zustand;
- Figur 2: schematisch eine weitere geschnittene Ansicht der Stechvorrichtung aus der Figur 1 in einem gespannten und einsatzbereiten Zustand;
- Figur 3: schematisch eine geschnittene Ansicht der Stechvorrichtung aus den Figuren 1 und 2 in einem Stechzustand;
- Figur 4: schematisch eine weitere geschnittene Ansicht der Stechvorrichtung aus den Figuren 1 bis 3 in einem Stechzustand;
- Figur 5: schematisch eine geschnittene Ansicht der Stechvorrichtung aus den Figuren 1 bis 4 nach einem Stechzustand;
- Figur 6: schematisch eine weitere geschnittene Ansicht der Stechvorrichtung aus den Figuren 1 bis 5 nach einem Stechzustand;
- Figur 7: schematisch eine geschnittene Ansicht der Stechvorrichtung aus den Figuren 1 bis 6 während eines Spannzustandes;
- Figur 8: schematisch eine weitere geschnittene Ansicht der Stechvorrichtung aus den Figuren 1 bis 7 während eines Spannzustandes;
- Figur 9: schematisch eine geschnittene Ansicht der Stechvorrichtung aus den Figuren 1 bis 8 in einem wieder gespannten und einsatzbereiten Zustand;
- Figur 10: schematisch eine perspektivische Ansicht eines Gehäuses der Stechvorrichtung aus den Figuren 1 bis 9;
- Figur 11: schematisch eine Ansicht eines zweiten Ausführungsbeispiels einer weiteren Stechvorrichtung mit einem axial ausgelenkten Spannkopfes einer Spanneinrichtung;
- Figur 12: schematisch eine Ansicht der weiteren Stechvorrichtung aus der Figur 11 mit zwei verschiedenen Spiralfederkonzepten;
- Figur 13: schematisch eine Ansicht hinsichtlich Mittel zum axialen Arretieren einer verlagerbaren Halteeinrichtung eines Stechmittels der weiteren Stechvorrichtung aus den Figuren 11 und 12;
- Figur 14: schematisch eine Ansicht hinsichtlich einer bogenförmigen Kurvenbahn der weiteren Stechvorrichtung aus den Figuren 11 bis 13; und
- Figur 15: schematisch eine Ansicht hinsichtlich einer von Mitteln zum Verrichten von Arbeit gespannten Antriebsfeder der weiteren Stechvorrichtung aus den Figuren 11 bis 14.

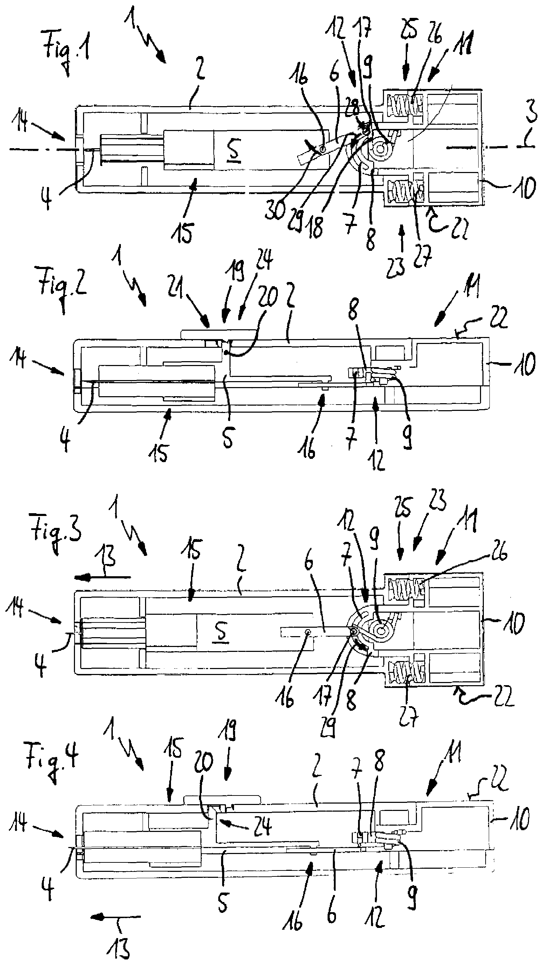

Die in den Figuren 1 bis 10 gezeigte Stechvorrichtung 1 weist ein Gehäuse 2 auf, in welchem entlang einer axialen Stechachse 3 (exemplarisch nur hinsichtlich der Figur 1 eingezeichnet und beziffert) im Wesentlichen ein axial verlagerbares Stechmittel 4, eine axial verlagerbare Halteeinrichtung 5, ein axial verlagerbares Koppelglied 6, eine axial verlagerbare bogenförmige Kurvenbahn 7, ein axial verlagerbares Steuerkurvenbahnelement 8, eine axial verlagerbare Antriebsfeder 9 und ein axial verlagerbarer Spannkopf 10 mit einem axial verschiebbaren Spannschlitten 11 angeordnet sind.

Insbesondere die Antriebsfeder 9, die bogenförmige Kurvenbahn 7, das Steuerkurvenbahnelement 8 und das Koppelglied 6 bilden Komponenten von Mittel 12 zum Antreiben der axial verlagerbaren Halteeinrichtung 5, mittels welchen das Stechmittel 4 eine translatorische Stechbewegung 13 (siehe Figuren 3 und 4) durch eine Öffnung 14 des Gehäuses 2 hindurch ausführen kann.

Die axial verlagerbare Halteeinrichtung 5 ist in Richtung der axialen Stechachse 3 mittels einer Linearführung 15 linear beweglich gelagert.

Das Koppelglied 6 ist auf Grund einer Lagerung 16 an der Halteeinrichtung 5 radial schwenkbar an der verlagerbaren Halteeinrichtung 5 gelagert, wobei das Koppelglied 6 einen Kurvenbahnfolger 17 in Gestalt eines Zapfens aufweist, der wiederum beweglich innerhalb einer geeigneten Kulisse (hier nicht beziffert) der bogenförmigen Kurvenbahn 7 gelagert ist.

Somit kann insbesondere mittels der Antriebsmittel 12 eine im Wesentlichen radial ausgeführte Bewegung eines ersten Schenkels 18 (der Übersichtlichkeit halber nur in der Figur 1 beziffert) der Antriebsfeder 9 in eine im Wesentlichen lineare Bewegung insbesondere der Halteeinrichtung 5 überführt werden.

An dem Gehäuse 2 der Stechvorrichtung 1 ist des Weiteren eine Auslöseeinrichtung 19 vorgesehen, mittels welcher die Stechbewegung 13 an der Stechvorrichtung 1 ausgelöst werden kann. Durch Betätigen der Auslöseeinrichtung 19 kann eine Rasteinrichtung 20 der verlagerbaren Halteeinrichtung 5 aus ihrer Rastposition 21 (siehe Figur 2) heraus bewegt werden, wodurch die Halteeinrichtung 5 speziell gemäß der Stechbewegung 13 translatorisch verlagert werden kann.

Die Stechvorrichtung 1 weist noch eine Spanneinrichtung 22 zum Spannen der Antriebsfeder 9 auf, wobei die Spanneinrichtung 22 einen Spannmechanismus 23 aufweist, dem einerseits Mittel 24 zum axialen Arretieren der verlagerbaren Halteeinrichtung 5 während des Spannens der Antriebsfeder 9 und andererseits Mittel 25 zum Verrichten von Arbeit während des Spannens der Antriebsfeder 9 zugeordnet werden können.

Die Mittel 24 zum Arretieren sind speziell in diesem ersten Ausführungsbeispiel baulich einfach direkt von der Rasteinrichtung 20 und korrespondierenden Komponenten der Auslöseeinrichtung 19 an der Stechvorrichtung 1 bereitgestellt werden. Es versteht sich, dass derartige Mittel 24 zum Arretieren auch an einer anderen Komponente der Stechvorrichtung 1 vorgesehen sein können.

Die Mittel 25 zum Verrichten von Arbeit sind hierbei von einer ersten Spiralfeder 26 und einer zweiten Spiralfeder 27 gebildet. Über die Spiralfedern 26 und 27 ist der Spannkopf 10 der Spanneinrichtung 22 federnd an dem Gehäuse 2 der Stechvorrichtung 1 gelagert.

Gemäß den Darstellungen nach den Figuren 1 und 2 befindet sich die Stechvorrichtung 1 in einem gespannten und betriebsbereiten Zustand. In diesem Zustand ist der Kurvenbahnfolger 17 in einer Startposition 28 an der bogenförmigen Kurvenbahn 7 positioniert. Sobald die Auslöseeinrichtung 19 betätigt wird, wird die Rasteinrichtung 20 freigegeben. Hierdurch kann eine Radialbewegung 29 des Kurvenbahnfolgers 17 entlang der bogenförmigen Kurvenbahn 7 erfolgen, woraufhin das Koppelglied 6 eine Schwenkbewegung 30 durchführt.

Durch die Radialbewegung 29 des Kurvenbahnfolgers 17 entlang der bogenförmigen Kurvenbahn 7 wird das Koppelglied 6 auch axial verlagert, wodurch die Halteeinrichtung 5 und das Stechmittel 4 die Stechbewegung 13 vollziehen.

Wie gemäß den Darstellungen der Figuren 3 und 4 gezeigt, befindet sich die Stechvorrichtung 1 dann in einem Stechzustand. In diesem Stechzustand ist das Stechmittel 4 aus der Öffnung 14 des Gehäuses 2 ausgetreten und der Kurvenbahnfolger 17 befindet sich in einer Mitte der zur Öffnung 14 hingewandten bogenförmigen Kurvenbahn 7. Durch eine fortwirkende Druckbeaufschlagung des Kurvenbahnfolgers 17 durch den ersten Schenkel 18 der Antriebsfeder 9 findet eine weitergehende Radialbewegung 29 des Kurvenbahnfolgers 17 statt (siehe Figur 3).

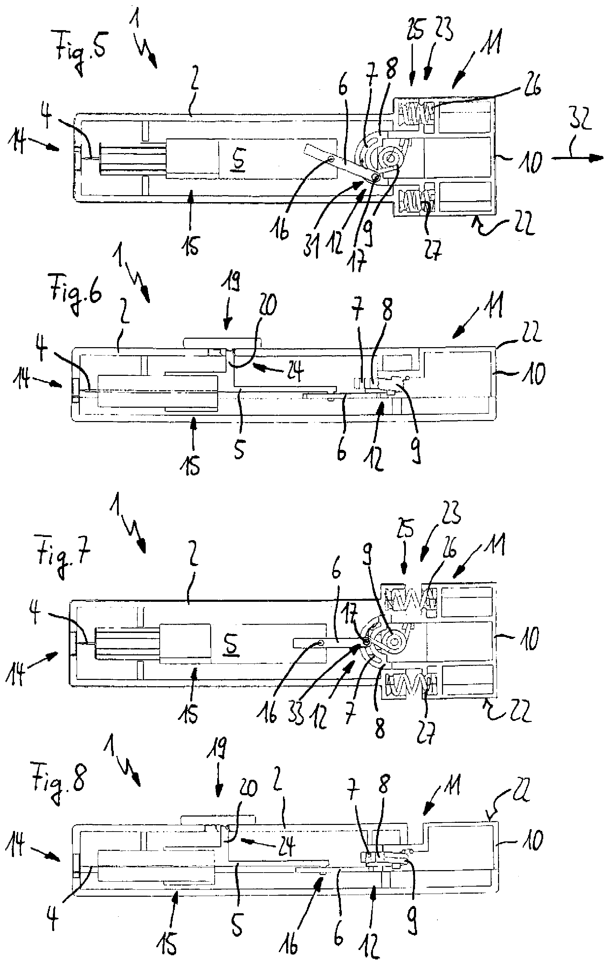

Ist der Kurvenbahnfolger 17 gemäß den Darstellungen nach den Figuren 5 und 6 in eine Endposition 31 verlagert, ist das Stechmittel 4 bereits wieder in das Gehäuse 2 zurückgezogen. Ein Stechvorgang ist damit beendet. In den Figuren 5 und 6 ist demnach ein Ruhezustand bzw. ein ungespannter Zustand der Stechvorrichtung 1 gezeigt. In diesem ungespannten Zustand hat der Kurvenbahnfolger 17 das andere Ende der bogenförmigen Kurvenbahn 7 erreicht und die Antriebsfeder 9 ist maximal aufgespannt und hierdurch im Wesentlichen entspannt.

Um die Antriebsfeder 9 wieder zu spannen, wird der Spannkopf 10 in Richtung einer Spannbewegung 32 (siehe Figur 5) axial ausgelenkt, wodurch die Anordnung der Antriebsmittel 12 in einen gestreckten Zustand hinein gezogen wird (siehe Figuren 7 und 8). Der Kurvenbahnfolger 17 befindet sich nun in einer Mittelposition 33 in der Mitte der bogenförmigen Kurvenbahn 7. Spätestens in diesem gestreckten Zustand wird die verlagerbare Halteeinrichtung 5 mittels der Mittel 24 zum Arretieren axial wieder festgelegt.

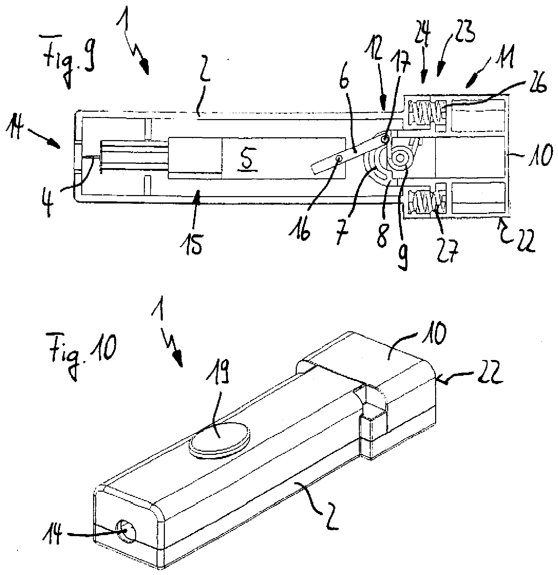

Wird der Spannkopf 10 losgelassen, wird er mittels der Zugkraft 34 der Spiralfedern 26 und 27 an das Gehäuse 2 herangezogen, wodurch sich insbesondere die bogenförmige Kurvenbahn 7 auf das Koppelglied 6 zu bewegt. Hierbei lenkt das Koppelglied 6 radial weiter aus und der Kurvenbahnfolger 17 kann wieder in die Startposition 28 bewegt werden (siehe Figur 9). Damit die Spiralfedern 26 und 27 die Antriebsfeder 9 derart spannen können, ist die Zugkraft 34 idealerweise größer als die Antriebskraft der Antriebsfeder 9 zu wählen.

Gemäß der Darstellung der Figur 9 befindet sich der Spannkopf 10 wieder in einem eingesteckten Zustand in dem Gehäuse 2 der Stechvorrichtung 1. Die Stechvorrichtung 1 befindet sich nun wieder in einem gespannten und betriebsbereiten Zustand, wie er auch hinsichtlich der Figuren 1 und 2 beschrieben wurde. Somit kann ein erneuter Stechvorgang erfolgen.

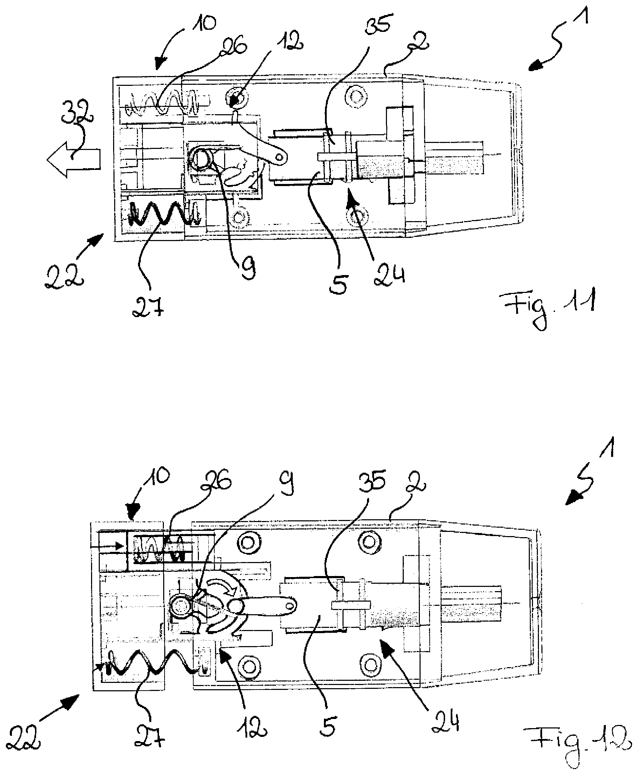

Weitere Details einer derartigen Spanneinrichtung 22 können den Figuren 11 bis 15 eines zweiten Ausführungsbeispiels einer weiteren Stechvorrichtung 1 entnommen werden.

Die Stechvorrichtung 1 des zweiten Ausführungsbeispiels weist ein Gehäuse 2 auf. Kopfseitig ist ein Spannkopf 10 vorgesehen, mittels welchem Mittel 12 zum Antreiben einer verlagerbaren Halteeinrichtung 5, insbesondere eine Antriebsfeder 9 der Antriebsmittel 12, gespannt werden können. Hierzu sind an dem Spannkopf 10 zwei Spiralfedern 26 und 27 vorgesehen, mit denen der Spannkopf 10 federnd an dem Gehäuse 2 gelagert sind. Wird der Spannkopf 10 mit einer Spannbewegung 32 von dem Gehäuse 2 wegbewegt, werden zum einen die Spiralfedern 26 und 27 gedehnt und zum anderen die Antriebsmittel 12 mit der Halteeinrichtung 5 gestreckt, indem diese Komponenten in Richtung der Spannbewegung 32 bewegt werden.

Hinsichtlich der Figur 12 sind schematisch zwei unterschiedliche Federkonzepte illustriert, welche hinsichtlich des Spannkopfes 10 an der Stechvorrichtung 1 realisierbar sind. Gemäß einer Ausführungsform ist die Spiralfeder 26 hierbei beispielhaft als Druckfeder ausgebildet. Die Spiralfeder 27 ist gemäß einer zweiten Ausführungsform als Zugfeder ausgebildet. Die Spiralfeder 27 ist endseitig dauerhaft befestigt. Gegebenenfalls lassen sich hierüber eine Spannkraft und ein Spannweg des Spannkopfes 10 einstellen.

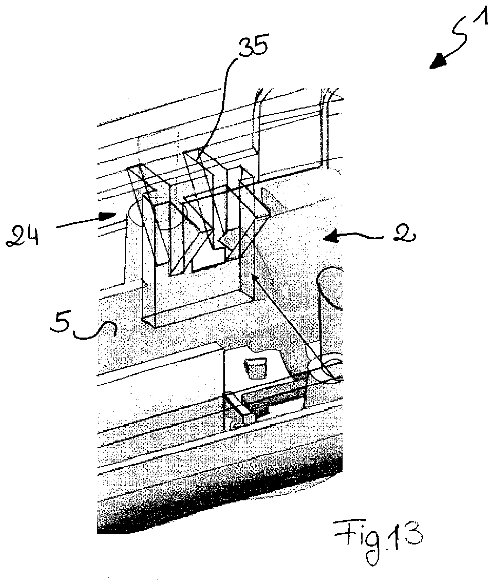

Insbesondere gemäß der Darstellung nach der Figur 13 sind noch alternative Mittel 24 zum axialen Arretieren der verlagerbaren Halteeinrichtung 5 gezeigt, welche radial an der verlagerbaren Halteeinrichtung 5 befestigt sind. Die Mittel 24 zum axialen Arretieren weisen Arretierrippen 35 (nur exemplarisch beziffert) auf, die mit geeigneten Haltenasen (hier nicht gezeigt) beispielsweise einer Auslöseeinrichtung der Stechvorrichtung 1 korrespondieren können. Mittels der Arretierrippen 35 kann insbesondere eine axiale Bewegung der Halteeinrichtung 5 während eines Spannens der Antriebsfeder 9 mittels des Spannkopfes 10 bzw. der Spiralfedern 26, 27 blockiert werden.

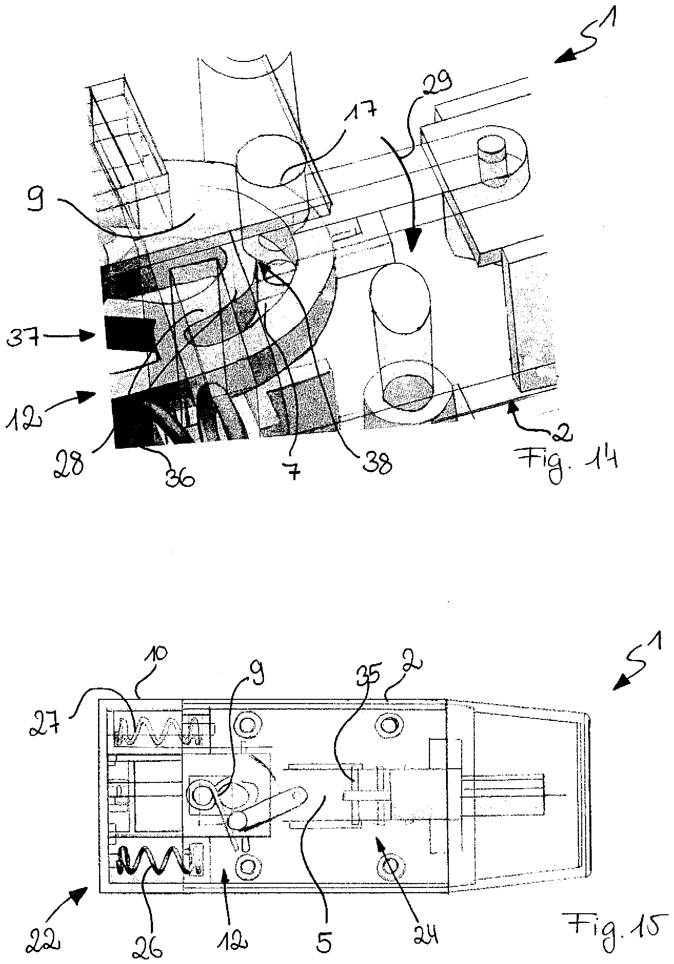

Bei der Darstellung nach der Figur 14 ist hinsichtlich der bogenförmigen Kurvenbahn 7 bezüglich einer Kulisse 36 erkennbar, dass die Kulisse 36 radial an einer Seite 37 eine Schräge 38 aufweist, so dass ein Kurvenbahnfolger 17 aus einer Mittelposition 33 der bogenförmigen Kurvenbahn 7 heraus automatisch mit einer Radialbewegung 29 wieder in eine Startposition 28 des Kurvenbahnfolgers 17 hinein verlagert wird, wenn die Antriebsfeder 9 mittels der Spiralfedern 26 und 27 neu gespannt wird, wie vorstehend hinsichtlich des ersten Ausführungsbeispiels bereits beschrieben. Die Schräge 38 bildet vorliegend einen in etwa geraden, linearen Abschnitt an der Kulisse 36 der bogenförmigen Kurvenbahn 7 aus, so dass der Kurvenbahnfolger 17 während des Spannens der Antriebsfeder 9 immer auf die richtige Seite 37 der bogenförmigen Kurvenbahn 7 gelenkt wird, an welcher der Kurvenbahnfolger 17 in der Startposition 28 positioniert werden kann. Die Seite 37 der bogenförmigen Kurvenbahn 7 setzt dem Kurvenbahnfolger 17 im Bereich der Schräge 38 weniger Widerstand entgegen, so dass der Kurvenbahnfolger 17 auch aus einem Stillstand heraus sich immer zuerst entlang der Schräge 38 bewegen wird.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Stechvorrichtung
- 2: Gehäuse
- 3: axiale Stechachse
- 4: Stechmittel
- 5: verlagerbare Halteeinrichtung
- 6: Koppelglied
- 7: bogenförmige Kurvenbahn
- 8: Steuerkurvenbahnelement
- 9: Antriebsfeder
- 10: Spannkopf
- 11: Spannschlitten
- 12: Mittel zum Antreiben der verlagerbaren Halteeinrichtung
- 13: Stechbewegung
- 14: Öffnung
- 15: Linearführung
- 16: Lagerung
- 17: Kurvenbahnfolger
- 18: erster Schenkel
- 19: Auslöseeinrichtung
- 20: Rasteinrichtung
- 21: Rastposition
- 22: Spanneinrichtung
- 23: Spannmechanismus
- 24: Mittel zum axialen Arretieren der verlagerbaren Halteeinrichtung
- 25: Mittel zum Verrichten von Arbeit
- 26: erste Spiralfeder
- 27: zweite Spiralfeder
- 28: Startposition
- 29: Radialbewegung
- 30: Schwenkbewegung
- 31: Endposition
- 32: Spannbewegung
- 33: Mittelposition
- 34: Zugkraft
- 35: Arretierrippen
- 36: Kulisse
- 37: Seite
- 38: Schräge

## Patentansprüche

1. Stechvorrichtung (1) für eine Blutprobenentnahme mit einer verlagerbaren Halteeinrichtung (5) für ein Stechmittel (4), mit Mitteln (12) zum Antreiben der verlagerbaren Halteeinrichtung (5), mit einer Auslöseeinrichtung (19) zum Auslösen einer Stechbewegung (13) des Stechmittels (4), bei welcher nach einem manuellen Betätigen der Auslöseeinrichtung (19) die verlagerbare Halteeinrichtung (5) mittels einer Federkraft einer Antriebsfeder (9) der Antriebsmittel (12) axial verlagert werden kann,
wobei eine Spanneinrichtung (22) zum Spannen der Antriebsfeder (9) vorgesehen ist, wobei die Spanneinrichtung (22) einen Spannmechanismus (23) umfassend ein Mittel (24) zum axialen Arretieren der verlagerbaren Halteeinrichtung (5) während des Spannens der Antriebsfeder (9) und umfassend ein Mittel (25) zum Verrichten von Arbeit während des Spannens der Antriebsfeder (9) aufweist,
wobei die Antriebsmittel (12) ein Koppelglied (6) zwischen der verlagerbaren Halteeinrichtung (5) und einem Steuerkurvenbahnelement (8) der Antriebsmittel (12) umfassen, wobei das Koppelglied (6) einen Kurvenbahnfolger (17), der innerhalb einer Kulisse des Steuerkurvenbahnelementes (8) angeordnet ist, umfasst
**dadurch gekennzeichnet, dass**
der Spannmechanismus einen axial verschiebbaren Spannschlitten (11) umfasst, wobei der axial verschiebbare Spannschlitten (11) einen Schiebesupport für ein Steuerkurvenelement (8) aufweist, wobei das Koppelglied (6) schwenkbar an der verlagerbaren Halteeinrichtung (5) gelagert ist und wobei der Kurvenbahnfolger (17) mittels einer Radialbewegung (29) des Kurvenbahnfolgers (17) entlang einer bogenförmigen Kurvenbahn des Steuerkurvenbahnelementes (8) bewegbar ist.

2. Stechvorrichtung (1) nach Anspruch 1, wobei der axial verschiebbare Spannschlitten (11) einen Schiebesupport für eine Antriebsfederlagerung aufweist.

3. Stechvorrichtung (1) nach einem der Ansprüche 1 oder 2, wobei die Mittel (25) zum Verrichten von Arbeit Federelemente, vorzugsweise zwei Spiralfedern (26, 27), aufweisen.

4. Stechvorrichtung (1) nach einem der Ansprüche 1 bis 3, wobei die Mittel (25) zum Verrichten von Arbeit einen Spannkopf (10) der Spanneinrichtung (22) federnd an einem Grundkörper (2) der Stechvorrichtung (1) lagern.

5. Stechvorrichtung (1) nach einem der Ansprüche 1 bis 4, wobei die Mittel (25) zum Verrichten von Arbeit eine Arbeitsfederkraft F_{D} und die Antriebsfeder (9) eine Antriebsfederkraft F_{S} aufweisen, wobei die Arbeitsfederkraft F_{D} größer ist als die Antriebsfederkraft F_{S}.

6. Stechvorrichtung (1) nach einem der Ansprüche 1 bis 5, wobei die Antriebsmittel (12) die Kurvenbahn umfassen, welche vorzugsweise als bogenförmige Kurvenbahn (7) ausgestaltet ist.

7. Stechvorrichtung (1) nach einem der Ansprüche 1 bis 6, wobei die Antriebsmittel (12) das Steuerkurvenbahnelement (8) umfassen.

8. Stechvorrichtung (1) nach einem der Ansprüche 1-7, wobei der Kurvenbahnfolger (17) einen Vorwärtstrieb aufweist, mittels welchem der Kurvenbahnfolger (17) entlang der Kurvenbahn in eine erste Richtung bewegt werden kann.

9. Stechvorrichtung (1) nach einem der Ansprüche 1-8, wobei der Kurvenbahnfolger (17) einen Rückwärtstrieb aufweist, mittels welchem der Kurvenbahnfolger (17) entlang der Kurvenbahn in eine zweite der ersten Richtung entgegengesetzten Richtung bewegt werden kann.

## Claims

1. Pricking apparatus (1) for taking a blood sample, comprising a moveable holding device (5) for a pricking means (4), means (12) for driving the moveable holding device (5), a trigger device (19) for triggering a pricking movement (13) of the pricking means (4), the moveable holding device (5) being axially moveable by means of a spring force of a drive spring (9) of the driving means (12) after manual activation of the trigger device (19),
a tensioning device (22) being provided for tensioning the drive spring (9), the tensioning device (22) having a tensioning mechanism (23) comprising a means (24) for axially locking the moveable holding device (5) during tensioning of the drive spring (9) and comprising a means (25) for performing work during tensioning of the drive spring (9),
the driving means (12) comprising a coupling member (6) between the moveable holding device (5) and a curved track control element (8) of the driving means (12), the coupling member (6) comprising a curved track follower (17) that is arranged inside a slot in the curved track control element (8),
**characterised in that**
the tensioning mechanism comprises an axially moveable tensioning slide (11), the axially moveable tensioning slide (11) comprising a sliding support for a curved control element (8), the coupling member (6) being pivotally mounted on the moveable holding device (5) and the curved track follower (17) being moveable along an arc-shaped curved track of the curved track control element (8) by means of a radial movement (29) of the curved track follower (17).

2. Pricking apparatus (1) according to claim 1,
wherein
the axially moveable tensioning slide (11) has a sliding support for a drive spring mounting.

3. Pricking apparatus (1) according to either claim 1 or claim 2,
wherein
the means (25) for performing work comprise spring elements, preferably two coil springs (26, 27).

4. Pricking apparatus (1) according to any of claims 1 to 3,
wherein
the means (25) for performing work supports a tensioning head (10) of the tensioning device (22) in a resilient manner on a main body (2) of the pricking apparatus (1).

5. Pricking apparatus (1) according to any of claims 1 to 4,
wherein
the means (25) for performing work have a working spring force FD and the drive spring (9) has a drive spring force FS, the working spring force FD being greater than the drive spring force FS.

6. Pricking apparatus (1) according to any of claims 1 to 5,
wherein
the driving means (12) comprise the curved track, which is preferably designed as an arc-shaped curved track (7).

7. Pricking apparatus (1) according to any of claims 1 to 6,
wherein
the driving means (12) comprise the curved track control element (8).

8. Pricking apparatus (1) according to any of claims 1 to 7,
wherein
the curved track follower (17) has a forward drive, by means of which the curved track follower (17) can be moved along the curved track in a first direction.

9. Pricking apparatus (1) according to any of claims 1 to 8,
wherein
the curved track follower (17) has a reverse drive, by means of which the curved track follower (17) can be moved along the curved track in a second direction counter to the first direction.

## Revendications

1. Dispositif de piqûre (1) pour le prélèvement d'un échantillon de sang avec un dispositif de support mobile (5) pour un moyen de piqûre (4), avec un moyen (12) pour entraîner le dispositif de maintien mobile (5), avec un dispositif de déclenchement (19) pour déclencher un mouvement de piqûre (13) du moyen de piqûre (4), dans lequel, après un actionnement manuel du dispositif de déclenchement (19), le dispositif de maintien mobile (5) peut être déplacé axialement grâce à la force élastique d'un ressort d'entraînement (9) du moyen d'entraînement (12),
un dispositif de serrage (22) étant prévu pour le serrage du ressort d'entraînement (9),
le dispositif de serrage (22) comprenant un mécanisme de serrage (23) muni d'un moyen (24) de blocage axial du dispositif de maintien mobile (5) pendant le serrage du ressort d'entraînement (9) et comprenant un moyen (25) de réalisation d'un travail pendant le serrage du ressort d'entraînement (9),
le moyen d'entraînement (12) comprenant un organe de couplage (6) entre le dispositif de maintien mobile (5) et l'élément de commande à piste courbe (8) du moyen d'entraînement (12), l'organe de couplage (6) comprenant un suiveur de piste courbe (17), disposé à l'intérieur d'une coulisse de l'élément de commande à piste courbe (8),
**caractérisé en ce que**
le mécanisme de blocage comprend un chariot de serrage mobile axialement (11),
le chariot de serrage mobile axialement (11) comportant un support coulissant pour l'élément de commande à piste courbe (8), l'organe de couplage (6) étant logé de manière pivotante au niveau du dispositif de maintien mobile (5), et le suiveur de piste courbe (17) pouvant être déplacé au moyen du mouvement radial (29) du suiveur de piste courbe (17) le long d'une piste courbe en forme d'arc de l'élément de commande à piste courbe (8).

2. Dispositif de piqûre (1) selon la revendication 1,
le chariot de serrage mobile axialement (11) comportant un support coulissant pour le logement du ressort d'entraînement.

3. Dispositif de piqûre (1) selon l'une des revendications 1 ou 2,
les moyens (25) de réalisation d'un travail comprenant des éléments élastiques, de préférence deux ressorts en spirale (26, 27)

4. Dispositif de piqûre (1) selon l'une des revendications 1 à 3,
les moyens (25) de réalisation d'un travail logeant une tête de serrage (10) du dispositif de serrage (22) de manière élastique au niveau du corps de base (2) du dispositif de piqûre (1).

5. Dispositif de piqûre (1) selon l'une des revendications 1 à 4,
les moyens (25) de réalisation d'un travail présentant une force élastique de travail FD et le ressort d'entraînement (9) présentant une force élastique de travail FS, la force élastique de travail FD étant supérieure à la force élastique de travail FS.

6. Dispositif de piqûre (1) selon l'une des revendications 1 à 5,
le moyen d'entraînement (12) comprenant la piste courbe qui est conçue de préférence sous la forme d'une piste courbe (7) en forme d'arc.

7. Dispositif de piqûre (1) selon l'une des revendications 1 à 6,
le moyen d'entraînement (12) comprenant l'élément de commande à piste courbe (8).

8. Dispositif de piqûre (1) selon l'une des revendications 1 à 7,
le suiveur de piste courbe (17) comprenant un entraînement vers l'avant à l'aide duquel le suiveur de piste courbe (17) peut être déplacé le long de la piste courbe dans une première direction.

9. Dispositif de piqûre (1) selon l'une des revendications 1 à 8,
le suiveur de piste courbe (17) comportant un entraînement vers l'arrière à l'aide duquel le suiveur de piste courbe (17) peut être déplacé le long de la piste courbe dans une deuxième direction opposée à la première direction.
